# EUROPEAN PATENT SPECIFICATION

(11) **EP 2 839 833 B1**
(45) Date of publication and mention of the grant of the patent: **13.06.2018**
(21) Application number: 13778344.5
(22) Date of filing: 18.04.2013
(51) Int. Cl.: A61K 9/00, A61K 31/519, A61K 31/045, A61K 31/4166, A61P 17/06, A61K 9/06, A61K 47/10, A61K 47/12, A61K 47/26, A61K 47/18, A61K 47/14, A61K 9/107, A61K 9/14

(54) **TOPICAL PHARMACEUTICAL COMPOSITION, METHOD FOR PRODUCING THE TOPICAL PHARMACEUTICAL COMPOSITION, USE OF THE TOPICAL PHARMACEUTICAL COMPOSITION AND METHOD FOR THE TOPICAL TREATMENT OF PSORIASIS, ATOPIC DERMATITIS OR CHRONIC ECZEMA**
TOPISCHE PHARMAZEUTISCHE ZUSAMMENSETZUNG, VERFAHREN ZUR HERSTELLUNG DER TOPISCHEN PHARMAZEUTISCHE ZUSAMMENSETZUNG, VERWENDUNG DER TOPISCHEN PHARMAZEUTISCHEN ZUSAMMENSETZUNG UND VERFAHREN ZUR TOPISCHEN BEHANDLUNG VON PSORIASIS, ATOPISCHER DERMATITIS ODER CHRONISCHEM EKZEM
COMPOSITION PHARMACEUTIQUE TOPIQUE, PROCÉDÉ DE PRODUCTION DE CETTE COMPOSITION PHARMACEUTIQUE TOPIQUE, UTILISATION DE CETTE COMPOSITION PHARMACEUTIQUE TOPIQUE ET MÉTHODE DE TRAITEMENT TOPIQUE DU PSORIASIS, DE LA DERMATITE ATOPIQUE OU DES ECZÉMAS CHRONIQUES

(30) Priority: 20.04.2012 BR 102012009350
(43) Date of publication of application: 25.02.2015
(73) Proprietor: Biolab Sanus Farmacêutica Ltda., CEP: 06767-220, Taboão da Serra - SP (BR)
(72) Inventor: JUNIOR, Dante Alario, CEP: 04551-000 São Paulo - SP (BR); PEREIRA, Jose Roberto da Costa, CEP: 04509-021 São Paulo - SP (BR)
(74) Representative: KATZAROV S.A.
(86) International application number: PCT/BR2013/000128
(87) International publication number: WO 2013/155584

(56) References cited:
- EP-A1- 0 247 900
- BR-A- 0 303 932
- CN-A- 102 451 301
- GB-A- 2 143 433
- ALVAREZ-FIGUEROA M J ET AL: "Transdermal delivery of methotrexate: iontophoretic delivery from hydrogels and passive delivery from microemulsions", INTERNATIONAL JOURNAL OF PHARMACEUTICS, ELSEVIER BV, NL, vol. 215, no. 1-2, 14 March 2001 (2001-03-14), pages 57-65, XP008122480, ISSN: 0378-5173, DOI: 10.1016/S0378-5173(00)00674-8 [retrieved on 2001-03-08]
- Niraj Kansara ET AL: "CONVENTIONAL TOPICAL TREATMENTS OF PSORIASIS", Pharmacologyonline, 1 January 2011 (2011-01-01), pages 397-407, XP055204722, Retrieved from the Internet: URL:http://www.unisa.it/uploads/4979/037.s inghal.pdf [retrieved on 2015-07-27]

## Description

### FIELD OF THE INVENTION

The present invention relates to a topical pharmaceutical composition comprising a combination of methotrexate, alpha bisabolol and allantoin; a process for producing the same and the use of the composition in the treatment of plaque psoriasis (psoriasis vulgaris), atopic dermatitis and chronic eczema. The composition of this invention can be used alone or in combination with other topical or systemic therapies. The present invention further discloses a process for producing the topical pharmaceutical composition.

### BACKGROUND OF THE INVENTION

Inflammation is the body's reaction to an infection or tissue injury. In an inflammatory process, the affected region becomes reddish and hot due to increased blood flow and other body fluids, which migrate into the inflamed area. The accumulation of cells from the immune system (white blood cells, macrophages and lymphocytes) also occur at the inflamed area. In some diseases, the inflammatory process can be destructive.

In autoimmune diseases and allergic reactions, inflammation is a major component. Autoimmune diseases are characterized by an immune response of the body against its own components identified as foreign bodies by the immune system. This results in an inflammatory reaction that takes several clinical characteristics, according to the tissue or system affected.

Psoriasis is a quite common chronic, autoimmune dermatitis, characterized by hyperproliferation of skin cells. The etiology of psoriasis is unknown, and emotional phenomena are often related to its emergence or aggravation, probably acting as triggering factors of a genetic predisposition to the disease. About 30% of people who suffer from psoriasis have a family history for this disease.

Psoriasis is a complex disease, its evolution or regression is unpredictable, and each case has its own development and severity, affecting both men and women in the age group between 20 and 40 years, and can arise at any stage of life. However, it occurs very frequently in white-skinned people, being rare in black, Indian and Asian, and does not exist among Eskimo.

In psoriasis, a severe local inflammatory response is triggered with formation of reddish plaques, wherein various inflammatory mediators may present great relevance in the development and aggravation of the disease, including the pro-inflammatory cytokines (such as interleukins IL-1, IL-8 and TNF-alpha). According to the identified state of the art (Umezawa, Y.; TNF-alpha inhibitors treatments for Psoriasis. Inflammation and Regeneration 2008, vol. 28, No. 01, 27-30) TNF-alpha is a pro-inflammatory cytokine with an important role in psoriasis pathology. High levels of TNF-alpha have been observed in psoriasis lesions, and the excess of TNF-alpha is directly related to the development, proliferation and maintenance of characteristic psoriasis plaques.

Psoriasis can present itself in the following types: plaque psoriasis (psoriasis vulgaris); inverted psoriasis; guttate psoriasis; psoriasis palmoplantaris; pustular psoriasis; erythrodermic psoriasis and psoriasis arthropathica. In most cases, only the skin is involved, and any impairment of other organs or systems is not observed. However, a small percentage may be associated with cases of arthritis.

Among the various types of classification for psoriasis, plaque psoriasis (psoriasis vulgaris) is the most common form, observed in 90% of patients, and is manifested by the formation of the erythemato-squamous plaques well-defined and of varying sizes.

Psoriasis is a chronic disease, and since it affects the skin, an external and visible organ, it has non negligible psychological effects. Various types of temporary reliefs are available and their effectiveness varies among patients. Topical and systemic treatments, or a combination thereof, are currently used for treating plaque psoriasis.

Among the systemic treatments, there can be cited the use of biological products administered parenterally (for example: Humira®, Remicade®, Enbrel®) and the use of active ingredients, such as methotrexate, cyclosporine and acitretin administered orally. These treatments are not effective for a definitive cure, only an improvement during treatment being observed.

Biological products currently available on the market are very expensive and invasive, whose application can only be made in an ambulatory environment, leading to a scarcely accessible and low adherence treatment.

Oral treatments are mostly toxic to the patient and only show improvements during the treatment period.

The mostly used topical treatments are products containing highly potent corticosteroids or calcipotriol (synthetic derivative of vitamin D) such treatments being, however, only palliative. Calcipotriol is very weak and only presents improvements in mild cases. Also, the prolonged or continuous use of topic corticosteroids on the same area causes thinning of the epidermis and alterations in the dermis. In most cases, the process is reversible with discontinuation of use, but the skin may take months to return to its normal state.

Methotrexate was developed in the decade of 1940 and is described in the US Patent No. 5.212.572 (American Cyanamid Company), and presents the following chemical structure:

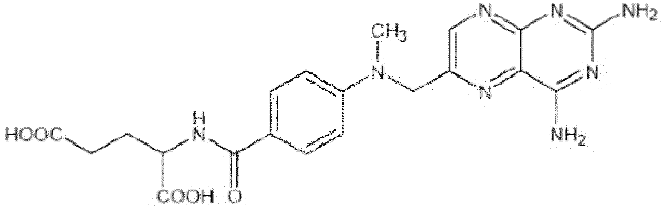

Methotrexate is known as an antimetabolite and it is usually administered orally, but it can also be administered intramuscularly, intravenously and intrathecally.

The action of methotrexate consists of the inhibition of DNA, RNA, timidinate and protein synthesis, acting specifically in S phase of the cell division cycle. Therefore, the growth of populations of cells that rapidly proliferate (malignant cells, epithelial cells in psoriasis) is more affected than the growth of skin cells and of most normal tissues. Methotrexate has a slight immunosuppressive activity and is widely used in the treatment of psoriasis by oral administration. However, the use of methotrexate by oral route only presents improvements during the period of treatment and is highly toxic to the patient. The adverse effects consist of bone marrow depression and epithelium injury of the gastrointestinal tract. Furthermore, when used in high dosages schemes, nephrotoxicity may occur. Hence, patients should be submitted to hematologic, hepatic and renal evaluations, and monitor symptoms that could diagnose when bone marrow depression occur.

The topical use of methotrexate in the treatment of psoriasis has been described in the state of the art, for example, in patents GB 1,153,767 (CASSENNE LAB SA) and GB 2,143,433 (PATEL HARIPRASAD MANIBHAI) and in scientific articles (Fry, L., McMinn, R.M.: Topical methotrexate in psoriasis.; Archives of dermatology, 1967, 96(5), 483-8, Journal code: 0372433; Van Scott, E.J., Reinertson, R.P.; Morphologic and physiologic effects of chemotherapeutic agents in psoriasis.; J Invest Drem 1959, 33:357 and Nurse, D.S.; Effect of Antimetabolites on Epidermal Strutures. Arch Derm 1963, 87:258). M. J. Alvarez-Figueroa et al., International Journal of Pharmaceutics 215 (2000) 57-65 disclose transdermal delivery of methotrexate from microemulsions. However, the results observed and discussed in these documents demonstrate controversies in relation to treatment efficacy (plaque reduction), and it was observed that the type of formulation used interferes in the results (Javadzadeh, Y., Hamishehkar, H.: Enhancing percutaneous delivery of methotrexate using different types of surfactants: Colloids and Surfaces B, Biointerfaces 82 2011, 422-426.).

The alpha bisbolol molecule is old and broadly known in the state of the art. Alpha bisabolol is a sesquiterpene alcohol found in the essential oil of many plants and has anti-inflammatory and soothing properties on the skin. Alpha bisabolol is known by the chemical name 1-methyl-4-(1,5-dimethyl-1-hydroxy-4(5)-hexenyl)-1-cyclohexene, has the molecular formula C₁₅H₂₆O, and possesses the following chemical structure:

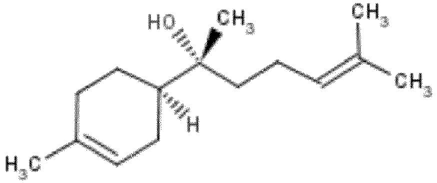

According to the state of the art, alpha bisabolol is a substance widely used in various cosmetic or pharmaceutical products, such as toothpaste, skin cleansers and body care products, sunscreens, antiperspirants, hair care products, products for treating acne and moisturizing creams; applied at concentrations that generally range from 0.05 to 0.30%.

The use of alpha-bisabolol in the treatment and/or reduction of patient troubles who suffer from dermatitis and psoriasis was described in the Brazilian patent BRPI0303932 (NEO BORDER LTDA) and Romania patent R0115938 (MIHAILESCU C. GHEORGHE OFFENBAC), but do not demonstrate efficacy. Additionally, there are no other clinical studies proving the effectiveness of alpha bisabolol compound in the treatment of psoriasis.

Allantoin is a chemical compound with molecular formula C₄H₆N₄O₃, chemical name 2,5-dioxo-4-imidazolidinyl urea and the following chemical structure:

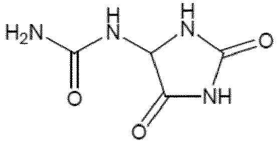

Allantoin is a compound widely used in skin care products and cosmetics, with recognized properties in cell renewal, keratolysis and improvement of skin moisturizing and hydration. The prior art states that allantoin is able to: promote cell proliferation accelerating the regeneration of injured skin; provide a quick epithelialization in injured or worn skin areas; reduce irritated states of skin and roughness; provide greater moisture retention capacity; and other anti-aesthetic signals, making the skin smooth and supple.

The use of allantoin in topical treatment of psoriasis was described in the US patent No. 3,043,745 (REED & CARNICK), which describes a lotion containing 2% of allantoin and tar extract for treating psoriasis, however, this patent does not present studies demonstrating the effectiveness of treatment. On the other hand, available prior art data reports that conducted clinical studies do not demonstrate the efficacy of isolated allantoin in the treatment of psoriasis (Herdensttam, C.G.: Allantoin in the treatment of psoriasis: a Double blind study: Acta Derm-vener 1959, 39:216; Young, E.: Allantoin in treatment of psoriasis: Dermatologica 1973, 147: 338-341.).

In topical compositions, the permeation of pharmaceutical and cosmetic active principles on the skin at molecular level is increasingly important to optimize the release system, transdermal release and bioavailability of active principles, and ultimately to help obtaining pharmaceutical compositions that have ideal permeability for the intended use, i.e. in which the active principles in the composition can easily permeate and reach only the epidermis (topical action) or deeper layers of the dermis, and be able to present systemic action.

Due to experimental difficulties to detect permeation of active principles through the skin, most studies use invasive methods, such as tape stripping and biopsies. Confocal Raman spectroscopy has recently proved to be an *in vivo* and ex *vivo* non-invasive measuring method that can provide detailed information, in real time, on the skin composition (for example: natural moisturizing factors - NMF, lipids, hydration level) and cutaneous permeation, based on known and well established patterns. This technique does not require sample preparation and can provide new data on the process of administering molecules to the skin.

According to prior art data, the skin is defined as a set of stratified tissues that cover the body and have structural components and metabolites that work in harmony with the environment to keep the organic balance. Representing 10 to 12% of body weight, it is characterized as the largest organ in the human body, with an area of approximately 1.5 m² and average thickness of 3 mm. It is composed of two main layers of tissue: dermis and epidermis. Dermis is the innermost layer of the skin that contains numerous many built-in structures and is highly vascularized. The great dermal vascularization allows drugs that reach this skin layer to achieve desired systemic levels or, in some cases, unwanted levels.

Epidermis is above the dermis (outermost layer of the skin), which act as a barrier that separates the external environment from internal homeostasis. Epidermis is specifically composed of the stratum corneum (SC) with depth ranging from 0 to 20 µm and viable epidermis with depth ranging from 21 to 40 µm.

Regarding the treatment of psoriasis, atopic dermatitis or chronic eczema, and diseases that affect the skin, it is of utmost importance that active principles, when applied topically, have greater concentration in the epidermis and, in some cases, such as in the use of active principles which have toxic adverse effects (methotrexate), it is very important that permeation is effective only at the epidermis level, and does not reach deeper layers of the dermis. Therefore, in accordance with this invention, the researchers developed a topical pharmaceutical composition in the form of an oil/water emulsion containing the combination of methotrexate, alpha bisabolol and allantoin, in which methotrexate is able to permeate through the skin and reach the epidermis showing effectiveness, but virtually without systemic absorption, eliminating nearly all observed adverse effects of its systemic use.

Although there are documents that describe the use of active principles in topical compositions for treating psoriasis, the results of clinical studies are inconclusive regarding the effectiveness of treatments with these active principles alone or in specific associations.

In face of the above scenario, available psoriasis treatments on the market, including topical ones, are not effective for a definitive cure, and prolonged use of some active principles has remarkable undesirable side effects. Thus, searching for new effective treatments for psoriasis, atopic dermatitis or chronic eczemas, the inventors developed a stable topical formulation, which presents a suitable profile of security and ease of administration, containing the triple combination of methotrexate, alpha bisabolol and allantoin. The composition developed by the present inventors is able to reduce inflammatory skin effect, even in the most severe cases, besides promoting skin hydration without the undesirable effects observed by absorption of methotrexate and the topical use of corticosteroids.

### SUMMARY OF THE INVENTION

The present invention aims at providing a topical pharmaceutical composition comprising a combination of methotrexate, alpha bisabolol and allantoin; and its use for treating inflammatory skin processes, more specifically for treating plaque psoriasis (psoriasis vulgaris), atopic dermatitis and chronic eczemas.

A first embodiment of the invention refers to a composition which is an oil/water emulsion at a ratio of 10/90 to 45/55 by weight, more preferably at a ratio of 15/85 to 35/65, comprising a combination of from 0.05% to 2% of micronized methotrexate monohydrate from 0.1 to 10% of alpha bisabolol, besides containing from 0.1 to 10% of allantoin and other pharmaceutically acceptable excipients, such as: acidifier agents, alkalinizing agents, antioxidant agents, chelating agents, preservative agents, wetting agents, emulsifying agents, surfactant agents, aqueous vehicles and oily vehicles.

Particularly, said embodiment of the invention relates to a topical composition comprising at least: (a) an aqueous phase consisting of 0.01 to 0.5% of an antioxidant agent; 0.01 to 1.0% of a chelating agent; 1 to 20% of a wetting agent; and 55 to 90% of aqueous vehicle; by total weight of the composition, and may optionally contain 0.5 to 3.0% of acidifying agent; 0.1 to 2.0% of alkalinizing agent; and 0.1 to 1.5% of preservative agent; by total weight of the composition, and (b) an oily phase consisting of 5 to 20% of an emulsifying agent; 0.5 to 5% of a surfactant agent; and 1.0 to 10.0% of an oily vehicle; by total weight of the composition.

In another aspect, the present invention relates to the topical composition for use in the treatment of psoriasis, atopic dermatitis and chronic eczemas.

In another aspect, the present invention relates to a process for producing a pharmaceutical composition for topical use comprising the combination of methotrexate, alpha bisabolol and allantoin.

### BRIEF DESCRIPTION OF THE FIGURES

Figure 1: skin fragment obtained from blepharoplasty.
Figure 2: Skin fragment with product application - test.
Figure 3: Graph of the result of evaluation of permeation of methotrexate at 2 h, 6 h and 24 h after application of formulation 2 (emulsion 20/80).
Figure 4: Graph of the result of evaluation of permeation of methotrexate at 2 h, 6 h and 24 h after application of formulation 1 (emulsion 30/70).

### DETAILED DESCRIPTION OF THE INVENTION

The present invention relates to a topical pharmaceutical composition comprising a combination of methotrexate, alpha bisabolol, allantoin and pharmaceutically acceptable excipients.

The allantoin comprised in the composition of the present invention can be used in a range from 0.1% to 10% of the total weight of the composition, being preferably present at 1% of the total weight of the composition.

The alpha bisabolol comprised in the composition of the present invention can be used in a range from 0.1% to 10% of the total weight of the composition, being preferably present at 2% of the total weight of the composition.

According to the present invention, the methotrexate comprised in the composition can be in the form of free base or pharmaceutically acceptable salts or hydrates thereof, the mono-hydrated form of methotrexate being preferentially used.

The methotrexate comprised in the composition has particles with defined size, preferably micronized, with 100% of methotrexate particles having a particle size with less than 30 µm of diameter, and more precisely particles with an average diameter size from 2 µm to 10 µm.

The methotrexate comprised in the composition may be used in a range from 0.05% to 2% of the total weight of the composition, being preferably present at about 0.26% of the total weight of the composition.

The pharmaceutically acceptable excipients comprised in the composition of the present invention include, but are not limited to, an acidifier agent, an alkalinizing agent, an antioxidant agent, a chelating agent, a preservative agent, a wetting agent, an emulsifying agent, a surfactant agent, an aqueous vehicle and an oily vehicle.

Examples of acidifying agents include, but are not limited to, lactic acid, citric acid, hydrochloric acid, phosphoric acid, tartaric acid and glycolic acid. The acidifying agent may be present from about 0.5% to about 3.0% of the total weight of the composition. Preferably, the acidifying agent is lactic acid and is present from 1% to 2% of the total weight of the composition.

Examples of alkalinizing agents include, but are not limited to, sodium hydroxide, potassium hydroxide, diethanolamine, triethanolamine and monoethanolamine. The alkalinizing agent may be present from 0.1% to 2.0% of the total weight of the composition. Preferably, the alkalinizing agent is sodium hydroxide and is present from 0.1% to 1% of the total weight of the composition.

Examples of antioxidant agents include, but are not limited to, butyl hydroxyanisole (BHA), butyl hydroxytoluene (BHT), ascorbyl palmitate, alpha-tocopherol (vitamin E), and mixtures thereof. The antioxidant agent may be present from 0.01% to 0.5% of the total weight of the composition. Preferably, the oxidizing agent is butyl hydroxyanisole (BHA) and is present from 0.01% to 0.03% of the total weight of the composition.

Examples of chelant agents include, but are not limited to, edetate disodium dihydrate (disodium EDTA dihydrate), disodium edetate (disodium EDTA), edetic acid (EDTA), calcium disodium edetate dihydrate, potassium edetate, sodium edetate, trisodium edetate and mixtures thereof. The chelant agent may be present from 0.01 to 1.0% of the total weight of the composition. Preferably, the chelant agent is edetate disodium dihydrate (disodium EDTA dihydrate) and is present from 0.08% to 0.16% of the total weight of the composition.

Examples of preservative agents include, but are not limited to, phenylpropanol, bronopol, butylparaben, ethylparaben, imidazolidinyl urea, methylparaben, phenoxyethanol and mixtures thereof. The preservative agent may be present from 0.1% to 1.5% of the total weight of the composition. Preferably, the preservative agent is phenylpropanol and is present in the range from 0.2% to 0.5% of the total weight of the composition.

Examples of wetting agent include, but are not limited to, glycerol (glycerin), propylene glycol, sorbitol, trehalose, triacetin, cyclomethicone and mixtures thereof. The wetting agent may be present in the composition from 1% to 20% of the total weight of the composition. Preferably, the wetting agent is glycerin and is present from 8% to 12% of the total weight of the composition.

Examples of emulsifying agents include, without limitation, cetostearyl alcohol, stearyl alcohol, cetyl alcohol, carbomer (acrylic acid polymer), poloxalene (poloxamer), self-emulsifying wax, polyethylene glycol stearate, ethylene glycol distearate, polyethylene glycol distearate, diethylene glycol monostearate, ethylene glycol monostearate, glyceryl monostearate, self-emulsifying glyceryl monostearate and mixtures thereof. The emulsifying agent may be present from about 5% to about 40% of the total weight of the composition. Preferably, the emulsifying agent is the mixture of cetostearyl alcohol and polyethylene glycol stearate and is preferably present in a range from 10 to 20% of the total weight of the composition.

Examples of surfactant agents include, but are not limited to, sorbitan monostearate, sorbitan tristearate, sorbitan stearate, sorbitan diisostearate, sorbitan dioleate, sorbitan monolaurate, sorbitan monooleate, sorbitan monopalmitate, sorbitan sesquioleate, sorbitan sesquistearate, sorbitan triisostearate, sorbitan trioleate, sorbitan tristearate, sodium lauryl sulfate, polysorbate, sodium docusate and mixtures thereof. The surfactant agent may be present in the range from 0.5% to 5% of the total weight of the composition. Preferably, the surfactant agent is the mixture of sorbitan monostearate and polysorbate, and is present in the range from 0.5% to 3% of the total weight of the composition.

Examples of oily vehicles include, but are not limited to, liquid petrolatum (liquid paraffin or mineral oil), olive oil, castor oil, corn oil, cottonseed oil, peanut oil, sesame oil, soybean oil, canola oil, polyethylene glycol, ethyl oleate, isopropyl myristate, isopropyl palmitate, medium chain triglycerides or mixtures thereof. The oily vehicle may be present in the range from 1% to 10% of the total weight of the composition. Preferably, the oily vehicle is liquid petrolatum and is present in the range from 1.5% to 3% of the total weight of the composition.

According to the present invention, the aqueous vehicle is water and may be present in the composition in the range from 45% to 90%, or more specifically in an amount sufficient to 100% (qsp) of the total weight of the composition.

The topical compositions of the present invention may be in the pharmaceutical forms of cream, cream-gel or lotion.

In another aspect of this invention, the topical formulation is an oil/water emulsion, in a ratio of 10/90 to 45/55 by weight. Preferably, the composition of this invention is an oil/water emulsion at a ratio of 15/85 to 35/65, comprising 0.05% to 2% of methotrexate, 0.1 to 10% of alpha bisabolol, from 0.1 to 10% of allantoin and pharmaceutically acceptable excipients.

According to the present invention, the topical composition in the form of oil/water emulsion consists of an oily phase and an aqueous phase.

The oily phase of the composition can be comprised by, but is not limited to, the following excipients: emulsifying agent, surfactant agent and oily vehicle.

The aqueous phase composition can be composed by, but is not limited to, the following excipients: antioxidant agent, chelant agent, wetting agent and aqueous vehicle, and it can further contain an acidifying agent, alkalizing agents and a preservative agent.

In a more preferred aspect, the aqueous phase of the composition consists of 0.01 to 0.5% of an antioxidant agent; 0.01 to 1.0% of a chelating agent; 1 to 20% of wetting agent; and 45 to 90% of aqueous vehicle, by total weight of the composition. Additionally, 0.5 to 3.0% of acidifying agent; 0.1 to 2.0% of alkalinizing agent; and 0.1 to 1.5% of preservative agent, by total weight of the composition, may be added to the aqueous phase.

In a more preferable aspect, the oily phase of the composition consists of 5 to 20% of emulsifying agent; 0.5 to 5% of surfactant agent; and 1.0 to 10.0% of oily vehicle; by total weight of the composition.

According to this invention, the topical pharmaceutical composition comprising a combination of methotrexate, alpha bisabolol and allantoin is used for treating inflammatory skin processes, more specifically for treating plaque psoriasis (psoriasis vulgaris), atopic dermatitis and chronic eczemas. And this composition can be used alone or in combination with other topical or systemic therapies.

Another important feature of the present invention relates to the fact that the methotrexate present in the topical composition has quick skin permeation and, after 24 hours, methotrexate residues are not detected anymore on the skin in the depths of interest, as described in example 3.

Another important aspect of the present invention refers to the process for producing a topical composition in the form of an oil/water emulsion as described in example 1, which comprises the steps of:
(a) Preparation of the oily phase, which comprises:
   (i) mixing the oily phase compounds; and
   (ii) heating the mixture to 60-75 °C under constant stirring, until complete fusion of the compounds;
(b) Preparation of the aqueous phase, which comprises:
   (i) mixing the aqueous phase compounds; and
   (ii) heating the mixture to 60-75 °C, under constant stirring;
(c) Incorporation of alpha bisabolol and allantoin to the oily phase;
(d) Cream formation, which comprises:
   (i) incorporating step "b" in step "a", and
   (ii) cooling the mass to 35-45 °C, under constant stirring; and
(e) Incorporation of methotrexate under constant stirring.

### EXAMPLES

### EXAMPLE 1: METHOD OF PRODUCTION OF THE COMPOSITION

The preparation of compositions of formula 1 or formula 2 (table 1) consists of the preparation of aqueous and oily phases, cream formation and the incorporation of methotrexate.

**Table 1: Formulation**

| **Components** | **Formula 1: O/W (30/70)** | **Formula 2: O/W (20/80)** |
|---|---|---|
| Active principles | | |
| Alpha bisabolol | 2.00% | 2.00% |
| Micronized Mono-hydrated Methotrexate | 0.26% | 0.26% |
| Allantoin | 1.00% | 1.00% |

| **Oily phase** | | |
|---|---|---|
| **Components** | **Formula 1: O/W (30/70)** | **Formula 2: O/W (20/80)** |
| Cetostearyl alcohol | 18.52% | 11.64% |
| PEG-40 stearate (polyethylene glycol) | 1.35% | 0.85% |
| Span 60 (sorbitan monostearate) | 1.35% | 0.85% |
| Polysorbate 80 | 2.70% | 1.70% |
| Liquid Paraffin | 2.70% | 1.70% |

| **Aqueous Phase** | | |
|---|---|---|
| Lactic Acid | 1.40% | 1.60% |
| Sodium hydroxide | 0.40% | 0.46% |
| Butyl hydroxyanisole | 0.02% | 0.02% |
| disodium EDTA dihydrate (edetate disodium dihydrate) | 0.10% | 0.11% |
| Phenylpropanol | 0.40% | 0.40% |
| Glycerin | 9.00% | 10.30% |
| Water | qsp 100% | qsp 100% |

### (a) Preparation of aqueous phase:

In an appropriate container:
- Mix water with sodium hydroxide and stir carefully until complete dissolution;
- Add lactic acid and stir until complete homogenization;
- Add BHA and EDTA and stir until complete dissolution;
- Add glycerin and fenilpropanol, and shake until total homogenization;
- Heat the mixture up to 70 °C; and
- Retain.

### (b) Preparation of oily phase:

- Add the excipients of the oily phase into an appropriate container;
- Heat the mixture up to 60 - 70 °C, under constant stirring, until complete homogenization of the components; and
- Keep the oily phase mixture at a temperature of 70 °C, under constant stirring; and
- Retain.

### (c) Incorporation of alpha bisabolol and allantoin:

- Add allantoin to the oily phase, under constant stirring, until complete homogenization;
- After, add alpha bisabolol to the oily phase, under constant stirring, until complete homogenization;

### (d) Cream formation:

- Incorporate the aqueous phase in the oily phase under constant agitation, at 70 °C, until complete homogenization;
- Cool down the mixture to 40 °C, under constant stirring, until the emulsion is formed;

### (e) Incorporation of methotrexate:

- Separate a portion of the preformed cream and retain;
- To the remainder of the preformed cream, add methotrexate slowly, until its complete incorporation;
- Add the cream containing methotrexate, the remainder of the preformed cream, under constant stirring, until its complete homogenization; and
- Cool down the final mixture to room temperature, under constant stirring.

### EXAMPLE 2: EVALUATION OF ANTI-INFLAMMATORY AND

### IMMUNOMODULATOR IN VITRO ACTIVITY

the effect of formulation 1 (described in example 1) was evaluated In this study, in the synthesis of inflammatory mediators: IL-1, IL-6, IL-8, IL-10, IL-12, prostaglandin E₂ (PGE₂), Leukotriene B4 (LBT4), interferon gamma (IFN-gamma) and tumor necrosis factor alpha (TNF-alpha) in a culture of human keratinocytes in basal condition and stimulated with bacterial lipopolysaccharides (LPS), to simulate the inflammatory response.

Human keratinocytes (Cascade Biologics, USA) were sown in 75 cm² bottles (Corning Inc, USA), cultured and expanded in a wet incubator to 37 °C in the presence of CO2, using a specific culture medium. When reaching confluence, cells were seeded in 24 well plates (Nunc, USA) for further incubation with non-toxic dilutions of formulation 1, described in example 1, and for evaluation of proposed mediators.

The cell cultures were incubated with various non-cytotoxic concentrations of formulation 1 described in example 1 (compositions containing the combination of methotrexate, alpha bisabolol and allantoin) and with control formulations containing the same base used for formulation 1, however, in this case, each control composition contained only one isolated active principle (methotrexate or alpha bisabolol). The evaluated concentrations were 0.0002; 0.0001; and 0.000025 and 0.00005% (w/v).

Cells were kept in touch with dilutions of the formulations and LPS (Sigma, USA) for 48 hours for further collection of the supernatant and the cell lysate. As a positive control of production of mediators, the cells were kept in touch only with LPS (Sigma, USA) in the same amount administered along with the formulations (samples), and as a basal control, cells alone were used, without the presence of LPS or formulations.

Quantification of inflammatory mediators (IL-1, IL-6, IL-8, IL-10, IL-12, IFN-gamma and TNF-alpha) was performed using a kit of enzyme-linked immunosorbent assay (sandwich ELISA) commercially available. And the quantification of PGE₂ and LBT4 mediators was performed by an ELISA competitive binding assay. The results were expressed in pg/mL, calculated from the values of references obtained with a standard curve constructed with known concentrations of the mediators of interest (table 2).

**Table 2: Production of inflammatory mediators**

| **IL-1** | | | |
|---|---|---|---|
| Basal | 191.69 + 23.66 | | |
| LPS | 665.60 + 57.17 | | |

| | | **Dilution: 0.0002** | **Dilution: 0.00005** |
|---|---|---|---|
| Alpha bisabolol | | 354.74 ± 37.03 | 313.43 ± 33.30 |
| Methotrexate | | 187.35 ± 26.22 | 291.69 ± 34.42 |
| Formulation 1 | | 106.91 ± 8.90 | 102.56 ± 18.03 |

| **TNF-alpha** | | | |
|---|---|---|---|
| Basal | 13.23 ± 1.93 | | |
| LPS | 22.54 ± 0.96 | | |

| | | **Dilution: 0.0002** | **Dilution: 0.00005** |
|---|---|---|---|
| Alpha bisabolol | | 8.00 ± 1.61 | 6.64 ± 0.32 |
| Methotrexate | | 4.82 ± 0.32 | 17.09 ± 1.61 |
| Formulation 1 | | 2.77 ± 1.29 | 6.64 ± 0.96 |

| **IL-8** | | | |
|---|---|---|---|
| Basal | 23.26 ± 9.81 | | |
| LPS | 102.13 ± 10.03 | | |

| | | **Dilution: 0.0002** | **Dilution: 0.00005** |
|---|---|---|---|
| Alpha bisabolol | | 28.90 ± 9.58 | 64.23 ± 13.00 |
| Methotrexate | | 19.39 ± 8.00 | 39.07 ± 7.53 |
| Formulation 1 | | 18.58 ± 4.56 | 38.42 ± 6.16 |

| **IL-12** | | | |
|---|---|---|---|
| Basal | 779.50 ± 35.35 | | |
| LPS | 1214.00 ± 88.39 | | |

| | | **Dilution: 0.0002** | **Dilution: 0.00005** |
|---|---|---|---|
| Alpha bisabolol | | 954.50 ± 70.71 | 979.50 ± 35.35 |
| Methotrexate | | 1004.50 ± 70.71 | 1042.00 ± 53.03 |
| Formulation 1 | | 967.00 ± 53.03 | 954.50 ± 35.35 |

| **IL-10** | | | |
|---|---|---|---|
| Basal | 28.11 ± 4.35 | | |
| LPS | 24.65 ± 1.08 | | |

| | | **Dilution: 0.0002** | **Dilution: 0.00005** |
|---|---|---|---|
| Alpha bisabolol | | 28.30 ± 1.36 | 27.15 ± 2.99 |
| Methotrexate | | 23.11 ± 1.08 | 29.08 ± 1.36 |
| Formulation 1 | | 30.62 ± 2.45 | 24.46 ± 3.54 |

| **LTB4** | | | |
|---|---|---|---|
| Basal | 638.62 ± 119.40 | | |
| LPS | 1361.59 ± 98.84 | | |

| | | **Dilution: 0.0002** | **Dilution: 0.00005** |
|---|---|---|---|
| Alpha bisabolol | | 648.55 ± 11.09 | 1083.83 ± 115.59 |
| Methotrexate | | 543.82 ± 78.75 | 1282.15 ± 174.81 |
| Formulation 1 | | **597.07 ± 106.60** | **510.06 ± 142.00** |

| | | | |
|---|---|---|---|
| Note: Values expressed in pg/mL and represent the mean **±** standard deviation. | | | |

According to the results obtained and described in Table 2, the inventors observed that there has been a sharp reduction in the production of IL-1, IL-8, TNF-alpha and LTB4 when compared to the production obtained by stimulation with LPS. It is important to emphasize that the production of these mediators was lower than the basal production (cells without stimuli), which may lead to the conclusion that formulation 1 has a great inhibitory activity of the production of IL-1, IL-8, TNF-alpha and LTB4. Another important fact observed was an increase in the production of IL-10, an important anti-inflammatory cytokine.

Thus, based on the data obtained and the activity of mediators tested, the inventors concluded that the sharp reduction observed for the production of IL-1, IL-8 and mainly of TNF-alpha, associated with the increase in the production of the anti-inflammatory cytokine IL-10, could be a strong indication of effectiveness of the composition in the treatment of psoriasis.

### EXAMPLE 3: EX-VIVO EVALUATION OF CUTANEOUS PERMEATION BY RAMAN METHOD

This study aimed at evaluating the cutaneous permeation of methotrexate present in the composition of this invention, more specifically of formulations 1 and 2 described in example 1.

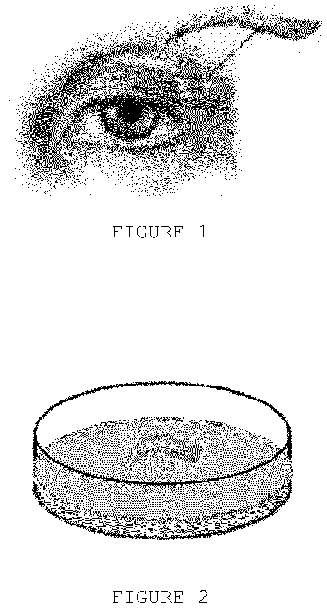

The evaluation of the permeation of methotrexate was performed through Raman method. The experiment consists of the steps:
- Obtainment of the fragment of human skin by blepharoplasty (figure 1);
- Hygienization of the skin fragment with alcohol 70% twice, followed by a bath in a specific culture medium - twice, to remove any residual alcohol 70%;
- Application of test product (formulations 1 and 2 - example 1): 2 mg/cm² = 6 mg of sample in an area of 3 cm². The application is made on the surface of the stratum corneum (figure 2). Both formulations described in example 1 were tested;
- The skin fragment containing the composition remained at rest for 2 hours, and then the excess of test product was mechanically taken off with the aid of pincers and gauzes;
- Readings were performed after 2 h, 6 h and 24 h from the application. At the reading intervals, the skin fragment was maintained in a CO₂ incubator (5 %) at 37 °C, immersed in culture medium. Evaluated reading parameters: depth of up to 40 µm (starting from the detection of skin surface); readings every 2 µm, after detection of the skin surface.

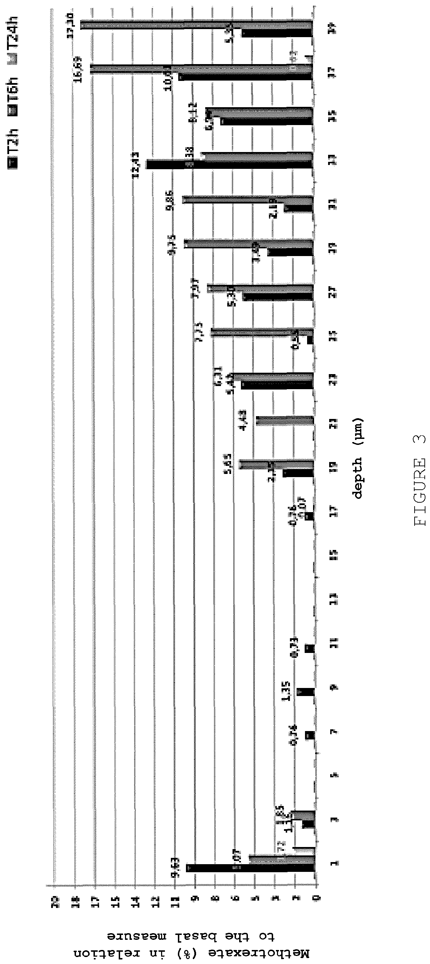

According to the obtained results (figure 3), it can be observed that, for formulation 2, after the first 2 hours of a single application, the presence of methotrexate is displayed at various depths, between 1 and 40 µm, which demonstrates a rapid permeation of methotrexate already in the first minutes of application. This initial increase, within the depth considered, identifies the significant presence of the product in the stratum corneum (SC) and viable epidermis. After the first two hours of application (T2h), the most prominent points with regards to the amount permeated were: 2 µm (9.63%), 33 µm (12.43%) and 37 µm (10.02%). Other interesting data which can be observed in this period (T2h), is that from the total amount of methotrexate applied to the skin (100%) only about 49.5% was found in the evaluated depth range (1 to 40 µm).

After 6 hours (T6h), as expected, taking into consideration the results obtained after 2 hours (T2h), methotrexate levels were reduced in relation to the first period in the upper layers, but started to increase in deeper layers, indicating a greater permeation with contact time. Methotrexate was significantly detected between the depths of 19 and 39 µm, which indicates the end of the stratum corneum (0 to 20 µm) to the viable epidermis (21 to 40 µm). Methotrexate peak was observed at 39 µm (17.30%). This depth corresponds to viable epidermis, which highlights that methotrexate reaches deeper layers of the epidermis, where it should necessarily be to perform the expected biological effect, i.e., to avoid uncontrolled cell proliferation, common in patients suffering from psoriasis. In this case, it is important to highlight that, in this evaluation period (T6h), more than 99% of the amount applied on the skin was found distributed along the depth measured in this study (1 to 40 µm). This indicates that, after a period of 6 hours, almost all methotrexate applied on the skin had permeated reaching only the epidermis, of which approximately 92% being distributed in the range of viable epidermis.

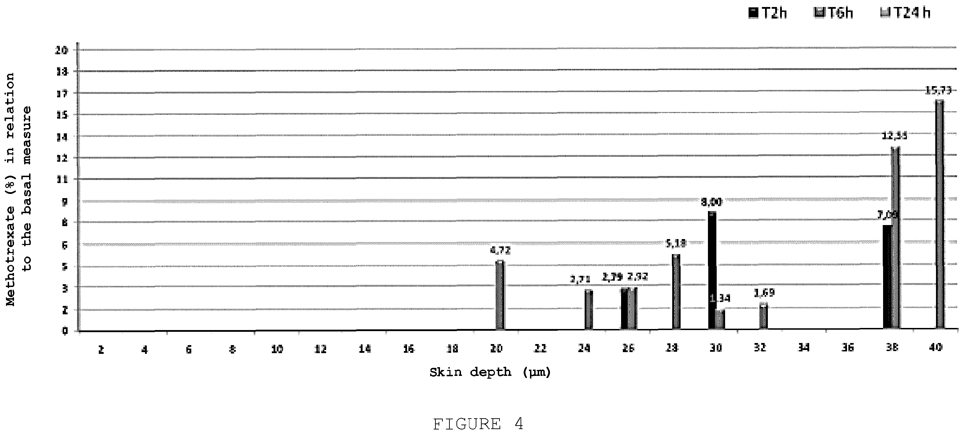

For formulation 1 described in example 1, according to the obtained results (figure 4), it can be observed that after the first 2 hours of a single application, the presence of methotrexate (marker for the formulation named formulation 1) is observed at various depths, between 2 and 40 µm, which demonstrates a fast permeation of methotrexate in the first minutes after application. This initial increase, within the depth considered, identifies the presence of the product in the viable epidermis. After the first two hours of application (T2h), the most prominent points with regards to the amount permeated were: 26 µm (2,79%), 30 µm (8,00%) and 38 µm (7,09%). In this period (T2h), from the total amount of methotrexate applied to the skin (100%) only about 17.9% was found in the depth range evaluated (1 to 40 µm).

After 6 hours (T6h), the methotrexate levels increase, taking into consideration the results obtained after 2 hours (T2h) in the deepest layers, indicating a greater permeation as the contact time increases. Methotrexate was significantly detected between the depths of 20 and 40 µm, which indicates the end of the stratum corneum (0 to 20 µm) to viable epidermis (21 to 40 µm). The methotrexate peak was observed at 40 µm (15,73%). This depth corresponds to the viable epidermis, which highlights that, in the 30/70 formulation, methotrexate also reaches deeper layers of the epidermis, where it should necessarily be to perform the expected biological effect. It is important to highlight that, in this evaluation period (T6h), about 47% of the total amount applied on the skin was distributed along the depth measured in this study (1 to 40 µm). This indicates that, after a period of 6 hours, less than 50.0% of total methotrexate applied on the skin had permeated, being distributed in the range of viable epidermis.

In order to evaluate the residence time of methotrexate in the skin, the evaluation of methotrexate levels after 24 hours from a single application of formulations 1 or 2 was carried out. According to the obtained results, it can be observed that, for formulation 2, after 24 hours of application, a residual level of methotrexate was detected only on the superficial layers of the skin, 1 µm (1.72%) (SC), and in the deeper layer of the skin, 39 µm (0.62%) (viable epidermis). Otherwise, no residual level of methotrexate after 24 hours was identified for formulation 1. These results demonstrate that, after 24 hours from a single application of methotrexate formulations 1 or 2, significant percentages of methotrexate in the skin, between 1 to 40 µm of depth, were no longer found.

Based on these results, it can be concluded that both formulations feature permeation of methotrexate to epidermis layers, preferably to the depth corresponding to the viable epidermis, which evidences that methotrexate reaches deeper layers of the epidermis, where it necessarily should be to perform the expected biological effect, i.e. avoid uncontrolled cell proliferation, which is common in patients suffering from psoriasis.

## Claims

1. TOPICAL PHARMACEUTICAL COMPOSITION, **characterized in that** it is an oil/water emulsion at a ratio of 10/90 to 45/55 by weight comprising a combination of:
(a) micronized methotrexate monohydrate from 0.05% to 2% by total weight of the composition;
(b) alpha bisabolol from 0.1 to 10% by total weight of the composition;
(c) allantoin from 0.1 to 10% by total weight of the composition; and
(d) pharmaceutically acceptable excipients.

2. TOPICAL PHARMACEUTICAL COMPOSITION, according to claim 1, **characterized in that** the methotrexate is in a micronized form with 100% of particles having a diameter of less than 30 µm.

3. TOPICAL PHARMACEUTICAL COMPOSITION, according to claim 2, **characterized in that** the methotrexate is in a micronized form with an average diameter of particles with preferred size in the range from 2 µm to 10 µm.

4. TOPICAL PHARMACEUTICAL COMPOSITION, according to claim 1, **characterized in that** the methotrexate is at a concentration from 0.1 to 0.5% by total weight of the composition; the alpha bisabolol is at a concentration from 1 to 4% by total weight of the composition and the allantoin is at a concentration from 0.5 to 3.0% by total weight of the composition.

5. TOPICAL PHARMACEUTICAL COMPOSITION, according to claim 1, **characterized in that** the pharmaceutically acceptable excipients are selected from the group consisting of: acidifying agent, alkalinizing agent, antioxidant agent, chelant agent, preservative agent, wetting agent, emulsifying agent, surfactant agent, aqueous vehicle and oily vehicle.

6. TOPICAL PHARMACEUTICAL COMPOSITION, according to any one of claims 1 to 5, **characterized in that** it comprises at least:
(a) an aqueous phase consisting of an antioxidant agent, a chelant agent, a wetting agent and an aqueous vehicle, and optionally containing an acidifying agent, an alkalinizing agent and a preservative agent; and
(b) an oily phase consisting of an emulsifying agent, a surfactant agent and an oily vehicle.

7. TOPICAL PHARMACEUTICAL COMPOSITION, according to claim 6, **characterized in that** it comprises at least:
(a) an aqueous phase consisting of 0.01 to 0.5% of antioxidant agent; 0.01 to 1.0% of chelant agent; 1 to 20% of wetting agent; and 45 to 80% of an aqueous vehicle, by total weight of the composition, optionally contains: 0.5 to 3.0% of acidifying agent; 0.1 to 2.0% of alkalinizing agent; and 0.1 to 1.5% of preservative agent; by total weight of the composition; and
(b) an oily phase consisting of 5 to 20% of emulsifying agent; 0.5 to 5% of surfactant agent; and 1.0 to 10.0% of oily vehicle; by total weight of the composition.

8. TOPICAL PHARMACEUTICAL COMPOSITION, according to claims 6 and 7, **characterized in that**:
(a) the antioxidant agent is selected from the group consisting of: butyl hydroxyanisole (BHA), butyl hydroxytoluene (BHT), ascorbyl palmitate, alpha-tocopherol (vitamin E) and mixtures thereof;
(b) the chelant agent is selected from the group consisting of edetate disodium dihydrate (disodium EDTA dihydrate), disodium edetate (disodium EDTA), edetic acid (EDTA), calcium disodium edetate dihydrate, potassium edetate, sodium edetate, trisodium edetate and mixtures thereof;
(c) the wetting agent is selected from the group consisting of: glycerol, propylene glycol, sorbitol, trehalose, triacetin, cyclomethicone and mixtures thereof;
(d) the acidifying agent is selected from the group consisting of lactic acid, citric acid, hydrochloric acid, phosphoric acid, tartaric acid and glycolic acid;
(e) the alkalinizing agent is selected from the group consisting of sodium hydroxide, potassium hydroxide, diethanolamine, triethanolamine and monoethanolamine;
(f) the preservative agent is selected from the group consisting of: phenylpropanol, bronopol, butylparaben, ethylparaben, imidazolidinyl urea, methylparaben, phenoxyethanol and mixtures thereof;
(g) the emulsifying agent is selected from the group consisting of: cetostearyl alcohol, stearyl alcohol, cetyl alcohol, carbomer (acrylic acid polymer), poloxalene (poloxamer), self-emulsifying wax, polyethylene glycol stearate, ethylene glycol distearate, polyethylene glycol distearate, diethylene glycol monostearate, ethylene glycol monostearate, glyceryl monostearate, self-emulsifiable glyceryl monostearate, propylene glycol monostearate and mixtures thereof;
(h) the surfactant agent is selected from the group consisting of: sorbitan monostearate, sorbitan tristearate, sorbitan stearate, sorbitan diisostearate, sorbitan dioleate, sorbitan monolaurate, sorbitan monooleate, sorbitan monopalmitate, sorbitan sesquioleate, sorbitan sesquistearate, sorbitan triisostearate, sorbitan trioleate, sodium lauryl sulfate, polysorbate, sodium docusate and mixtures thereof;
(i) the aqueous vehicle is water; and
(j) the oily vehicle is selected from the group consisting of: liquid petrolatum (liquid paraffin or mineral oil), olive oil, castor oil, corn oil, cottonseed oil, peanut oil, sesame oil, soybean oil, canola oil, polyethylene glycol, ethyl oleate, isopropyl myristate, isopropyl palmitate, medium chain triglycerides or mixtures thereof.

9. TOPICAL PHARMACEUTICAL COMPOSITION, according to claims 1 to 8, **characterized in that** it is in the pharmaceutical form of a cream, cream-gel or lotion.

10. TOPICAL PHARMACEUTICAL COMPOSITION, according to claim 7, **characterized in that** it comprises at least:
(a) an aqueous phase consisting of 0.01 to 0.5% of butyl hydroxyanisole (BHA); 0.01 to 1.0% of edetate disodium dihydrate (disodium EDTA dihydrate); 1 to 20% of glycerol; and 45 to 80% of water; by total weight of the composition, and optionally contains 0.5 to 3.0% of lactic acid; 0.1 to 2.0% of sodium hydroxide; and 0.1 to 1.5% of phenylpropanol; by total weight of the composition, and
(b) an oily phase consisting of 5 to 20% of cetostearyl alcohol and polyethylene glycol stearate; 0.5 to 5% of sorbitan monostearate and polysorbate; and 1.0 to 10.0% of liquid petrolatum (liquid paraffin or mineral oil); by total weight of the composition.

11. PROCESS FOR PRODUCING A TOPICAL PHARMACEUTICAL COMPOSITION, as defined in any one of claims 1 to 10, comprising the steps of:
(a) preparation of the oily phase consisting of: (i) mixing the oily phase compounds; and (ii) heating the mixture to 60-75 °C under constant stirring, until complete fusion of compounds;
(b) preparation of the aqueous phase consisting of: (i) mixing the aqueous phase compounds; and (ii) heating the mixture to 60-75 °C under constant stirring;
(c) incorporation of allantoin and alpha-bisabolol in the oily phase;
(d) formation of a cream consisting of (i) incorporation of the aqueous phase, of step "b", in the oily phase, of step "a", and (ii) cooling the mass to 35-45 °C, under constant stirring; and
(e) Incorporation of methotrexate.

12. PROCESS FOR PRODUCING A TOPICAL PHARMACEUTICAL COMPOSITION, according to claim 11, **characterized in that** the step of methotrexate incorporation comprises the following steps:
(i) in a portion of the preformed cream, add slowly the methotrexate and stirring until full incorporation of methotrexate;
(ii) addition of the cream portion containing the methotrexate to the remainder of the cream; and
(iii) cool down of the mixture to room temperature by stirring.

13. TOPICAL PHARMACEUTICAL COMPOSITION according to any one of claims 1 to 10 for use in the treatment of psoriasis, atopic dermatitis or chronic eczemas.

## Patentansprüche

1. TOPISCHE PHARMAZEUTISCHE ZUSAMMENSETZUNG, **dadurch gekennzeichnet, dass** sie eine Öl/Wasser-Emulsion in einem Verhältnis von 10/90 bis 45/55, bezogen auf das Gewicht ist, mit einer Kombination von:
(a) micronisiertem Methotrexat-Monohydrat, in einer Konzentration von 0,05 bis 2% des Gesamtgewichts der Zusammensetzung;
(b) alpha-Bisabolol, in einer Konzentration von 0,1 bis 10% des Gesamtgewichts der Zusammensetzung;
(c) Allantoin, in einer Konzentration von 0,1 bis 10% des Gesamtgewichts der Zusammensetzung; und
(d) pharmazeutisch annehmbaren Hilfsstoffen.

2. TOPISCHE PHARMAZEUTISCHE ZUSAMMENSETZUNG gemäss Anspruch 1, **dadurch gekennzeichnet, dass** das Methotrexat in einer mikronisierten Form vorliegt, wobei 100% der Teilchen einen Durchmesser von weniger als 30µm aufweisen.

3. TOPISCHE PHARMAZEUTISCHE ZUSAMMENSETZUNG gemäss Anspruch 2, **dadurch gekennzeichnet, dass** das Methotrexat in einer mikronisierten Form vorliegt, mit einem durchschnittlichen Durchmesser der Teilchen mit einer bevorzugten Grösse im Bereich von 2µm bis 10µm.

4. TOPISCHE PHARMAZEUTISCHE ZUSAMMENSETZUNG gemäss Anspruch 1, **dadurch gekennzeichnet, dass** das Methotrexat in einer Konzentration von 0,1 bis 0,5% des Gesamtgewichts der Zusammensetzung vorliegt; das alpha-Bisabolol in einer Konzentration von 1 bis 4% des Gesamtgewichts der Zusammensetzung vorliegt; und das Allantoin in einer Konzentration von 0,5 bis 3,0% des Gesamtgewichts der Zusammensetzung vorliegt.

5. TOPISCHE PHARMAZEUTISCHE ZUSAMMENSETZUNG gemäss Anspruch 1, **dadurch gekennzeichnet, dass** die pharmazeutisch annehmbaren Hilfsstoffe ausgewählt sind aus der Gruppe bestehend aus: Säuerungsmittel, Alkalisierungsmittel, Antioxidationsmittel, Chelierungsmittel, Konservierungsmittel, Netzmittel, Emulgator, Tensid, wässriges Vehikel und öliges Vehikel.

6. TOPISCHE PHARMAZEUTISCHE ZUSAMMENSETZUNG gemäss irgendeinem der Ansprüche 1 bis 5, **dadurch gekennzeichnet, dass** sie mindestens umfasst:
(a) eine wässrige Phase, bestehend aus Antioxidationsmittel, Chelierungsmittel, Netzmittel und wässrigem Träger, und gegebenenfalls enthaltend: Säuerungsmittel, Alkalisierungsmittel und Konservierungsmittel; und
(b) eine Ölphase, bestehend aus Emulgator, Tensid und öligem Träger.

7. TOPISCHE PHARMAZEUTISCHE ZUSAMMENSETZUNG gemäss Anspruch 6, **dadurch gekennzeichnet, dass** sie mindestens umfasst:
(a) eine wässrige Phase, bestehend aus 0,01 bis 0,5% Antioxidationsmittel; 0,01 bis 1,0% Chelierungsmittel; 1 bis 20% Netzmittel; und 45 bis 80% wässrigem Vehikel, bezogen auf das Gesamtgewicht der Zusammensetzung, und gegebenenfalls enthaltend: 0,5 bis 3,0% Säuerungsmittel; 0,1 bis 2,0% Alkalisierungsmittel; und 0,1 bis 1,5% Konservierungsmittel; bezogen auf das Gesamtgewicht der Zusammensetzung; und
(b) eine Ölphase, bestehend aus 5 bis 20% Emulgator; 0,5 bis 5% Tensid; und 1,0 bis 10,0% öligem Vehikel; bezogen auf das Gesamtgewicht der Zusammensetzung.

8. TOPISCHE PHARMAZEUTISCHE ZUSAMMENSETZUNG gemäss den Ansprüchen 6 und 7, **dadurch gekennzeichnet, dass**
(a) das Antioxidationsmittel ausgewählt ist aus der Gruppe bestehend aus: Butylhydroxyanisol (BHA), Butylhydroxytoluol (BHT), Ascorbylpalmitat, alpha-Tocopherol (Vitamin E) und Mischungen davon;
(b) das Chelierungsmittel ausgewählt ist aus: Edetat-Dinatriumdihydrat (Dinatrium-EDTA-Dihydrat), Dinatriumedetat (Dinatrium-EDTA), Edetinsäure (EDTA), Calcium-Dinatriumedetat-Dihydrat, Kalium Edetat, Natriumedetat, Trinatriumedetat und Mischungen davon;
(c) das Benetzungsmittel ausgewählt ist aus der Gruppe bestehend aus: Glycerin, Propylenglycol, Sorbitol, Trehalose, Triacetin, Cyclomethicon und Mischungen davon;
(d) das Säuerungsmittel ausgewählt ist aus der Gruppe bestehend aus Milchsäure, Zitronensäure, Salzsäure, Phosphorsäure, Weinsäure und Glykolsäure;
(e) das Alkalisierungsmittel ausgewählt ist aus der Gruppe bestehend aus: Natriumhydroxid, Kaliumhydroxid, Diethanolamin, Triethanolamin und Monoethanolamin
(f) das Konservierungsmittel ausgewählt ist aus der Gruppe bestehend aus: Phenylpropanol, Bronopol, Butylparaben, Ethylparaben, Imidazolidinylharnstoff, Methylparaben, Phenoxyethanol und Mischungen davon:
(g) der Emulgator ausgewählt ist aus der Gruppe bestehend aus: Cetostearylalkohol, Stearylalkohol, Cetylalkohol, Carbomer (Acrylsäurepolymer), Poloxalen (Poloxamer), selbstemulgierendem Wachs, Polyethylenglykol Stearat, Ethylenglycoldistearat, Polyethylenglycoldistearat, Diethylenglycolmonostearat, Ethylenglycolmonostearat, Glycerylmonostearat, selbstemulgierbares Glycerylmonostearat, Propylenglycolmonostearat und Mischungen davon;
(h) das oberflächenaktive Mittel ausgewählt ist aus der Gruppe bestehend aus: Sorbitanmonostearat, Sorbitantristearat, Sorbitanstearat, Sorbitandiisostearat, Sorbitandioleat, Sorbitanmonolaurat, Sorbitanmonooleat, Sorbitanmonopalmitat, Sorbitansesquioleat, Sorbitan Sesquistearat, Sorbitantriisostearat, Sorbitantrioleat, Sorbitantristearat, Natriumlaurylsulfat, Polysorbat, Natriumdocusat und Mischungen davon;
(i) das wässrige Vehikel Wasser ist; und
(j) das ölige Vehikel ausgewählt ist aus der Gruppe bestehend aus flüssigem Petrolatum (flüssiges Paraffin oder Mineralöl), Olivenöl, Rizinusöl, Maisöl, Baumwollsamenöl, Erdnussöl, Sesamöl, Sojabohnenöl, Canolaöl, Polyethylenglykol, Ethyloleat, Isopropylmyristat, Isopropylpalmitat,mittelkettigen Triglyceriden oder Mischungen davon.

9. TOPISCHE PHARMAZEUTISCHE ZUSAMMENSETZUNG gemäss den Ansprüchen 1 bis 8, **dadurch gekennzeichnet, dass** sie in der pharmazeutischen Form einer Creme, eines Creme-Gels oder einer Lotion vorliegt.

10. TOPISCHE PHARMAZEUTISCHE ZUSAMMENSETZUNG gemäss Anspruch 7, **dadurch gekennzeichnet, dass** sie mindestens umfasst:
(a) eine wässrige Phase, bestehend aus 0,01 bis 0,5% Butylhydroxyanisol (BHA); 0,01 bis 1,0% Edetat-Dinatriumdihydrat (Dinatrium-EDTA-Dihydrat); 1 bis 20% Glycerin; und 45 bis 80% Wasser; durch das Gesamtgewicht der Zusammensetzung, und gegebenenfalls enthaltend: 0,5 bis 3,0% Milchsäure; 0,1 bis 2,0% Natriumhydroxid; und 0,1 bis 1,5% Phenylpropanol; bezogen auf das Gesamtgewicht der Zusammensetzung; und
(b) eine Ölphase, bestehend aus 5 bis 20% Cetostearylalkohol und Polyethylenglycolstearat; 0,5 bis 5% Sorbitanmonostearat und Polysorbat; und 1,0 bis 10,0% flüssiges Petrolatum (flüssiges Paraffin oder Mineralöl); bezogen auf das Gesamtgewicht der Zusammensetzung.

11. VERFAHREN ZUR HERSTELLUNG EINER TOPISCHEN PHARMAZEUTISCHEN ZUSAMMENSETZUNG, wie in irgendeinem der Ansprüche 1 bis 10 definiert, mit folgenden Schritten:
(a) Herstellung der Ölphase, bestehend aus: (i) Mischen der Verbindungen der öligen Phase; und (ii) Erhitzen der Mischung auf 60 bis 75°C unter konstantem Rühren bis zur vollständigen Verschmelzung der Verbindungen;
(b) Herstellung der wässrigen Phase, bestehend aus: (i) Mischen der wässrigen Phasenverbindungen; und (ii) Erhitzen der Mischung auf 60 bis 75°C unter konstantem Rühren;
(c) Einbau von Allantoin und Alpha-Bisabolol in die Ölphase;
(d) Bildung einer Creme, bestehend aus (i) Einbringen der wässrigen Phase von Schritt "b" in die Ölphase von Schritt "a" und (ii) Abkühlen der Masse auf 35 bis 45°C unter konstantem Rühren; und
(e) Einbau von Methotrexat.

12. VERFAHREN ZUR HERSTELLUNG EINER TOPISCHEN PHARMAZEUTISCHEN ZUSAMMENSETZUNG gemäss Anspruch 11, **dadurch gekennzeichnet, dass** der Schritt des Methotrexat-Einbaus die folgenden Schritte umfasst:
(i) in einem Teil der vorgeformten Creme das Methotrexat langsam zugeben und bis zum vollständigen Einbau von Methotrexat rühren;
(ii) Zugabe des Rahmanteils, der das Methotrexat enthält, zu dem Rest der Creme; und
(iii) Abkühlen der Mischung auf Raumtemperatur durch Rühren.

13. TOPISCHE PHARMAZEUTISCHE ZUSAMMENSETZUNG gemäss irgendeinem der Ansprüche 1 bis 10, zur Verwendung in der Behandlung von Psoriasis, atopischer Dermatitis oder chronischen Ekzemen.

## Revendications

1. COMPOSITION PHARMACEUTIQUE TOPIQUE, **caractérisée en ce qu'**elle est une émulsion huile/eau dans une proportion de 10/90 à 45/55 en poids, comprenant une combinaison de:
(a) méthotrexate monohydraté micronisé à raison de 0.05% à 2% par poids total de la composition;
(b) alpha bisabolol à raison de 0.1 à 10% par poids total de la composition;
(c) allantoïne; à raison de 0.1 à 10% par poids total de la composition ; et
(d) des excipients pharmaceutiquement acceptables.

2. COMPOSITION PHARMACEUTIQUE TOPIQUE, selon la revendication 1, **caractérisée en ce que** le méthotrexate est sous une forme micronisée, avec 100% de particules ayant un diamètre inférieur à 30µm.

3. COMPOSITION PHARMACEUTIQUE TOPIQUE, selon la revendication 2, **caractérisée en ce que** le méthotrexate est sous une forme micronisée avec un diamètre moyen de particules de taille préférée dans la fourchette de 2µm à 10µm.

4. COMPOSITION PHARMACEUTIQUE TOPIQUE, selon la revendication 1, **caractérisée en ce que** le méthotrexate est à une concentration de 0,1 à 0,05% en poids total de la composition ; l'alpha bisabolol est à une concentration de 1 à 4% en poids total de la composition ; et l'allantoïne est à une concentration de 0,5 à 3.0% en poids total de la composition.

5. COMPOSITION PHARMACEUTIQUE TOPIQUE, selon la revendication 1, **caractérisée en ce que** les excipients pharmaceutiquement acceptables sont choisis dans le groupe constitué par: agent acidifiant, agent alcalinisant, agent antioxydant, agent chélateur, agent conservateur, agent mouillant, agent émulsifiant, agent tensioactif, véhicule aqueux et véhicule huileux.

6. COMPOSITION PHARMACEUTIQUE TOPIQUE, selon l'une quelconque des revendications 1 à 5, **caractérisée en ce qu'**elle comprend au moins:
(a) une phase aqueuse consistant en un agent antioxydant, un agent chélateur, un agent mouillant et un véhicule aqueux, et comprenant optionnellement un agent acidifiant, un agent alcalinisant et un agent conservateur; et
(b) une phase huileuse consistant en un agent émulsifiant, un agent tensioactif et un véhicule huileux.

7. COMPOSITION PHARMACEUTIQUE TOPIQUE, selon la revendication 6, **CARACTERISEE en ce qu'**elle comprend au moins :
(a) une phase aqueuse constituée de 0,01 à 0,5% d'agent antioxydant; 0,01 à 1,0% d'agent chélatant; 1 à 20% d'agent mouillant; et de 45 à 80% de véhicule aqueux, en poids total de la composition, contenant optionnellement 0,5 à 3,0% d'agent acidifiant; 0,1 à 2,0% d'agent alcalinisant; et 0,1 à 1,5% d'agent de conservation; en poids total de la composition ; et
(b) une phase huileuse constituée de 5 à 20% d'agent émulsionnant; 0,5 à 5% d'agent tensioactif; et 1,0 à 10,0% de véhicule huileux; en poids total de la composition.

8. COMPOSITION PHARMACEUTIQUE TOPIQUE, selon les revendications 6 et 7, **caractérisée en ce que** :
(a) l'agent antioxydant est choisi dans le groupe constitué par: butyl hydroxyanisole (BHA), butyl hydroxytoluène (BHT), palmitate d'ascorbyle, alpha-tocophérol (vitamine E) et leurs mélanges ;
(b) l'agent chélateur est choisi dans le groupe constitué par: édétate disodique dihydraté (EDTA disodique dihydraté), édétate disodique (EDTA disodique), acide édétique (EDTA), édétate disodique dihydraté dihydraté, potassium édétate, édétate de sodium, édétate trisodique et leurs mélanges
(c) l'agent mouillant est choisi dans le groupe constitué par: glycérol, propylèneglycol, sorbitol, tréhalose, triacétine, cyclométhicone et leurs mélanges
(d) l'agent acidifiant est choisi dans le groupe constitué par: l'acide lactique, l'acide citrique, l'acide chlorhydrique, l'acide phosphorique, l'acide tartrique et l'acide glycolique ;
(e) l'agent alcalinisant est choisi dans le groupe constitué par: l'hydroxyde de sodium, l'hydroxyde de potassium, la diéthanolamine, la triéthanolamine et la monoéthanolamine ;
(f) l'agent conservateur est choisi dans le groupe constitué par: phénylpropanol, bronopol, butylparabène, éthylparabène, imidazolidinylurée, méthylparabène, phénoxyéthanol et leurs mélanges
(g) l'émulsifiant est choisi dans le groupe constitué par: alcool cétostéarylique, alcool stéarylique, alcool cétylique, carbomère (polymère d'acide acrylique), poloxalène (poloxamère), cire auto-émulsionnante, polyéthylèneglycol le stéarate, le distéarate d'éthylèneglycol, le distéarate de polyéthylèneglycol, le monostéarate de diéthylèneglycol, le monostéarate d'éthylèneglycol, le monostéarate de glycéryle, le monostéarate de glycéryle auto-émulsifiable, le monostéarate de propylèneglycol et leurs mélanges
(h) l'agent tensioactif est choisi parmi: monostéarate de sorbitan, tristéarate de sorbitan, stéarate de sorbitan, diisostéarate de sorbitan, dioléate de sorbitan, monolaurate de sorbitan, monooléate de sorbitane, monopalmitate de sorbitan, sorbitan sesquioléate, sorbitan sesquistéarate, triisostéarate de sorbitan, trioléate de sorbitan, laurylsulfate de sodium, polysorbate, docusate de sodium et leurs mélanges ;
(i) le véhicule aqueux est de l'eau ; et
(j) le véhicule huileux est choisi dans le groupe constitué par: pétrolatum liquide (huile de paraffine ou huile minérale), huile d'olive, huile de ricin, huile de maïs, huile de coton, huile de cacahuète, huile de sésame, huile de soja, huile de canola, polyéthylèneglycol, oléate d'éthyle, myristate d'isopropyle, palmitate d'isopropyle, les triglycérides à chaîne moyenne ou leurs mélanges.

9. COMPOSITION PHARMACEUTIQUE TOPIQUE, selon les revendications 1 à 8, **caractérisée en ce qu'**elle est sous la forme pharmaceutique d'une crème, d'un gel-crème ou d'une lotion.

10. COMPOSITION PHARMACEUTIQUE TOPIQUE, selon la revendication 7, **caractérisée en ce qu'**elle comprend au moins:
(a) une phase aqueuse constituée de 0,01 à 0,5% de butyl hydroxyanisole (BHA); 0,01 à 1,0% d'édétate disodique dihydraté (EDTA disodique dihydraté); 1 à 20% de glycérol; et 45 à 80% d'eau; en poids total de la composition, et optionnellement contenant 0,5 à 3,0% d'acide lactique ; 0,1 à 2,0% d'hydroxide de sodium ; et 0,1 à 1,5% de phénylpropanol; en poids total de la composition ; et
(b) une phase huileuse constituée de 5 à 20% d'alcool cétostéarylique et de stéarate de polyéthylèneglycol; 0,5 à 5% de monostéarate de sorbitan et de polysorbate; et 1,0 à 10,0% d'huile de vaseline (huile de paraffine liquide ou huile minérale); en poids total de la composition.

11. PROCEDE DE PRODUCTION D'UNE COMPOSITION PHARMACEUTIQUE TOPIQUE, telle que définie dans l'une quelconque des revendications 1 à 10, caractérisé en comprenant les étapes consistant à:
(a) préparer la phase huileuse consistant à: (i) mélanger les composés de la phase huileuse; et (ii) chauffer le mélange à 60-75°C sous agitation constante jusqu'à la fusion complète des composés;
(b) préparation de la phase aqueuse consistant à: (i) mélanger les composés en phase aqueuse; et (ii) chauffer le mélange à 60-75°C sous agitation constante;
(c) incorporation d'allantoïne et d'alpha-bisabolol dans la phase huileuse;
(d) formation de crème consistant en (i) l'incorporation de la phase aqueuse de l'étape "b" dans la phase huileuse de l'étape "a" et (ii) le refroidissement de la masse à 35-45°C, sous constante en remuant; et
(e) incorporation de méthotrexate.

12. PROCEDE DE PRODUCTION D'UNE COMPOSITION PHARMACEUTIQUE TOPIQUE, selon la revendication 11, **caractérisé en ce que** l'étape d'incorporation de méthotrexate comprend les étapes suivantes:
(i) dans une partie de la crème préformée, ajouter lentement le méthotrexate et bien agiter jusqu'à incorporation complète de méthotrexate;
(ii) l'addition de la partie crème contenant le méthotrexate au reste de la crème; et (iii) refroidir le mélange à la température ambiante en agitant.

13. COMPOSITION PHARMACEUTIQUE TOPIQUE, selon l'une quelconque des revendications 1 à 10 à usage dans le traitement du psoriasis, de la dermatite atopique ou des eczémas chroniques.
